Europäisches Patentamt

European Patent Office

Office européen des brevets

(11) Publication number: **0 289 232**
**A2**

## (12) EUROPEAN PATENT APPLICATION

(21) Application number: 88303666.7

(22) Date of filing: 22.04.88

(51) Int. Cl.4: **C07C 41/06 , C07C 43/04**

(30) Priority: 29.04.87 US 43729

(43) Date of publication of application:
02.11.88 Bulletin 88/44

(84) Designated Contracting States:
**BE DE FR GB IT NL**

(71) Applicant: **MOBIL OIL CORPORATION**
**150 East 42nd Street**
**New York New York 10017(US)**

(72) Inventor: **Harandi, Mohsen Nadimi**
**12 Catbird Court**
**Lawrenceville New Jersey 08648(US)**
Inventor: **Owen, Hartley**
**5 Riverview Terrace**
**Belle Mead New Jersey 08502(US)**
Inventor: **Smyth, Sean Connon**
**132-B 6th Street Manhattan Beach**
**Los Angeles, California 90266(US)**

(74) Representative: **Colmer, Stephen Gary**
**Patent Department c/o Mobil Services**
**Company Limited Mobil Court 3 Clements**
**Inn**
**London WC2A 2EB(GB)**

(54) **Feedstock dewatering and etherification of crude methanol.**

(57) An improved process for reacting crude aqueous methanol feedstock with isoolefinic hydrocarbons to produce $C_5^+$ methyl isoalkyl ethers, which comprises: contacting the aqueous methanol feedstock with a liquid hydrocarbon extractant rich in $C_4^+$ isoalkene under liquid extraction conditions; recovering an aqueous phase containing the major amount of water introduced with the feedstock; recovering an organic extract phase comprising the hydrocarbon extractant and a major amount of methanol introduced in the feedstock; and reacting the extracted methanol and $C_4^+$ isoalkene in contact with an acid etherification catalyst under catalytic reaction conditions to produce ether product.

EP 0 289 232 A2

# FEEDSTOCK DEWATERING AND ETHERIFICATION OF CRUDE METHANOL

This invention relates to conversion of crude methanol or the like to lower methyl tertiary alkyl ethers. In particular, this invention relates to an integrated system for converting crude methanol to valuable products by etherifying lower branched olefins, such as $C_4$-$C_5$ isoolefins. It is known that isobutylene may be reacted with methanol over an acidic catalyst to provide methyl tertiary butyl ether (MTBE) and isoamylenes may be reacted with methanol over an acidic catalyst to produce tertiary-amyl methyl ether (TAME). Those ethers having the formula $CH_3$-O-R, where R is a tertiary alkyl radical, are particularly useful as octane improvers for liquid fuels, especially gasoline.

Increased demand for high octane gasolines blended with lower aliphatic alkyl ethers as octane boosters and supplementary fuels has created a significant demand for tertiary alkylethers especially the $C_5$ to $C_7$ methyl alkyl ethers, such as methyl tertiary butyl ether (MTBE) and tertiary amyl methyl ether (TAME). Methanol may be readily obtained from coal by gasification to synthesis gas and conversion of the synthesis gas to methanol by well-established industrial processes. As an alternative, the methanol may be obtained from natural gas by other conventional processes, such as steam reforming or partial oxidation to make the intermediate syngas. Crude methanol from such processes usually contains a significant amount of water, usually in the range of 4 to 20 wt%, however, the present invention is useful for removing water in lesser amounts or greater. The catalyst employed is preferably an ion exchange resin in the hydrogen form. Substantially any acidic catalyst may be employed with varying degrees of success. That is, acidic solid catalysts may be used; such as, sulfonic resins, phosphoric acid modified kieselguhr, silica alumina and acid zeolites.

The present invention provides a process for reacting crude aqueous methanol feedstock with isoolefinic hydrocarbons to produce $C_5^+$ methyl tertiary alkyl ethers. The improvement herein comprises: contacting the aqueous methanol feedstock with a liquid hydrocarbon extractant rich in isoalkene under liquid extraction conditions; recovering an aqueous phase containing the major amount of water introduced with the feedstock; recovering an organic extract phase comprising the hydrocarbon extractant and a major amount of methanol introduced in the feedstock; and reacting the extracted methanol and isoalkene in contact with an acid etherification catalyst under catalytic reaction conditions to produce ether product. In addition, methods and apparatus are provided for distilling the aqueous raffinate stream to recover methanol and residual hydrocarbon for reaction in the etherification reactor. In a preferred embodiment of the invention the reaction effluent with a portion of fractionated raffinate water recovered from the distillation step to wash methanol from the effluent and thereby provide a product stream rich in methyl tertiary alkyl ether; and further fractionating methanol-rich wash water together with raffinate.

In the drawings,

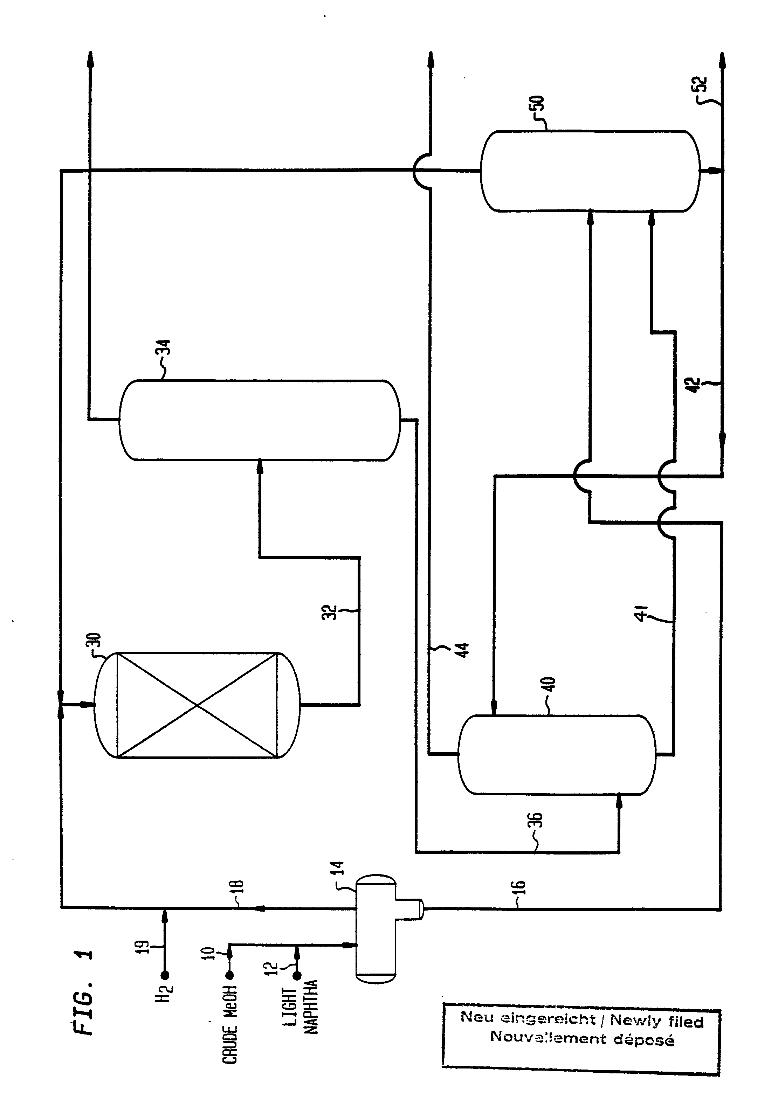

FIG. 1 is a schematic etherification process flowsheet depicting the present invention;

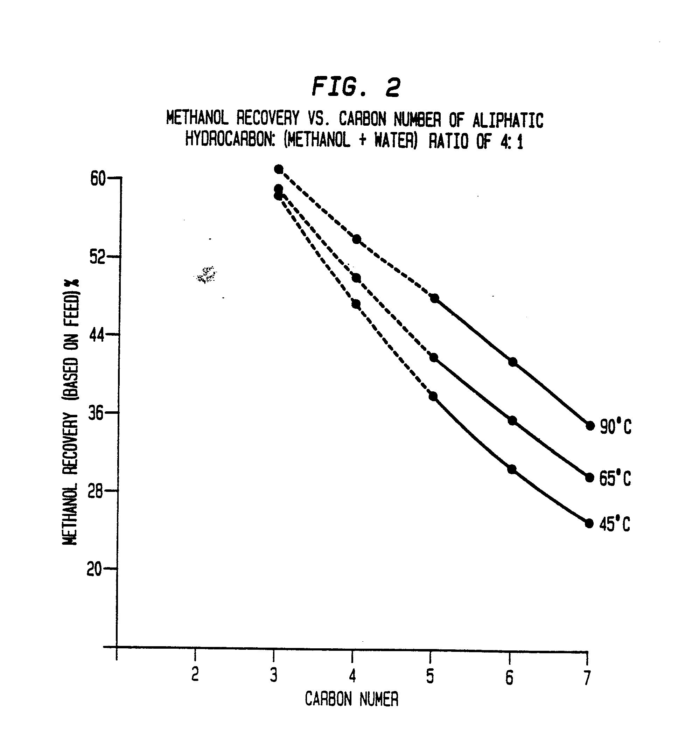

FIG. 2 is a graphic plot of methanol recovery vs. carbon number of aliphatic extractant, showing extraction temperature as parameter; and

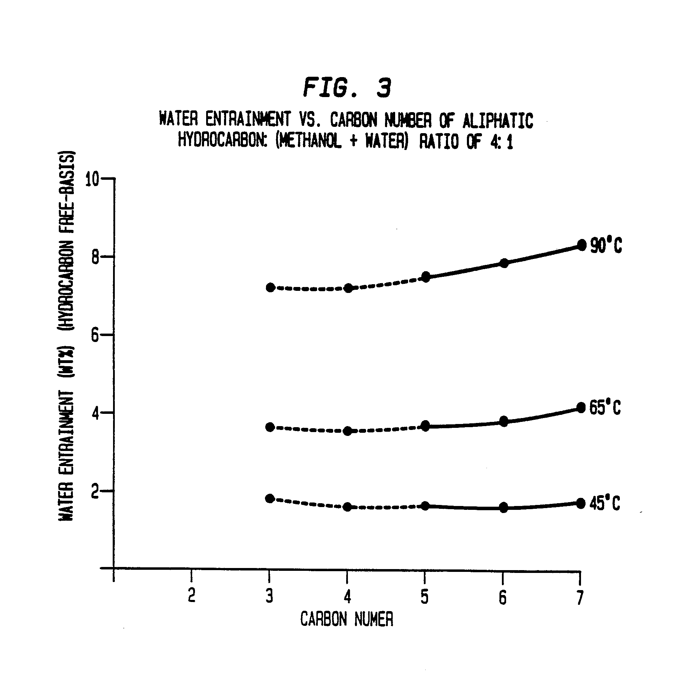

FIG. 3 is a corresponding graphic plot showing water entrainment in the extract phase for $C_3$-$C_7$ aliphatics.

Typical feedstock materials for etherification reactions include olefinic streams, such as fluidized catalytic cracking (FCC) light naphtha and butenes rich in isoolefins. These aliphatic streams are produced in petroleum refineries by catalytic cracking of gas oil or the like. The crude methanol commercially available from syngas processes may contain, for instance 4 to 17 wt% water, which must be removed, preferrably to a methanol purity of about 99.8 wt%. It has been found that more than 75% of crude feedstock methanol can be recovered by liquid extraction with light olefinic liquid extractant, such as butenes and $C_5^+$ light olefinic naphtha. The typical feed ratio range is 5 to 20 parts hydrocarbon extractant per part by volume of methanol.

Typical equipment according to the present invention includes a continuous feedstock separation and etherification reactor system for converting crude methanol oxygenate feedstock and isoolefin to methyl t-alkyl ether, wherein the unit operation apparatus includes: a) extractor means for contacting crude alcohol feedstock liquid containing a minor amount of water with a liquid hydrocarbon extraction stream under extraction conditions favorable to selective extraction of oxygenate, thereby providing an extract liquid stream rich in oxygenate and an aqueous raffinate stream lean in oxygenate; b) catalytic reactor means operatively connected for contacting the extract stream in a catalytic reaction zone with acid etherification catalyst in an etherification reaction zone under process conditions to convert methanol to ether; c) raffinate separator means for distilling the aqueous raffinate stream to recover methanol and residual hydrocarbon for reaction in the catalytic reactor; d) effluent washer means for contacting etherification reaction effluent with a portion of fractionated raffinate water recovered from the raffinate separator to wash methanol from the effluent and thereby provide a product stream rich in methyl isoalkyl ether; and e) means for passing methanol-rich wash water from the effluent washer for codistillation with raffinate.

Referring to the drawing, a continuous stream of crude methanol (MeOH) feedstock is introduced via conduit 10 with a stream of hydrocarbon liquid extractant introduced via conduit 12 to a top inlet of extraction separation unit 14. These streams are contacted under liquid extraction conditions to provide an aqueous raffinate phase. An aqueous stream containing a major amount of the water present in the crude feedstock is withdrawn via conduit 16. The lighter organic extract phase containing hydrocarbon extraction solvent and the major amount of feedstock methanol is recovered from extraction unit 14 via conduit 18, combined with hydrogen from line 19 and introduced under temperature and process conditions suitable for conversion of methanol in contact with etherification catalyst in reactor system 30. From the reactor system 30, the effluent product stream leaves via line 32 to an optional effluent stabilizer tower 34 where light gas, including hydrogen is removed from the system.

Stabilizer tower bottoms comprise unreacted $C_4^+$ hydrocarbons and methanol which are passed via conduit 36 to effluent washer vessel 40, where it is contacted with wash water introduced via line 42 for extraction of unreacted methanol from the etherol product stream 44, rich in ethers. The aqueous raffinate stream 16 consists essentially of waters, partititioned methanol and a trace of hydrocarbon. This may be recovered and fractionated along with the water wash tower 40 bottom 41 in fractionation tower 50. The bottom of tower 50 provides a wastewater stream 52 and wash water stream 42.

Alternatively, hydrocarbon feed containing $C_4$ and $C_5^+$ components can be processed by debutanizing the etherification reactor effluent. This technique can provide a condensed $C_4^-$ stream to be used in the water wash tower wherein methanol is separated from unreacted $C_4$ components.


EXTRACTION UNIT OPERATION


The typical preferred crude feedstock material is methanol containing 4 to 17% by weight water. The extraction contact unit may be a stirred multi-stage vertical extraction column adapted for continuous operation at elevated pressure. Any suitable extraction equipment may be employed, including concurrent, cross-current or single stage contactors, wherein the liquid methanol feedstock is intimately contacted with a substantially immiscible liquid hydrocarbon solvent, which may be a mixture of $C_4^+$ aliphatic components including lower alkanes, n-alkenes or relatively pure isoalkenes, such as isobutylene, etc. This unit operation is described in Kirk-Othmer Encyclopedia of Chemical Technology (Third Ed.)₁ 1980, pp.672-721. Other equipment for extraction is disclosed in US Patents 4,349,415 (DeFilipi et al) and 4,626,415 (Tabak). The methanol extraction step can be performed advantageously in a countercurrent multistage design, such as a simple packed column, rotating disc column, agitated column with baffles or mesh, or a series of single stage mixers and settlers.

As an example of typical methanol extraction with FCC light naphtha in a liquid-liquid contact and separation unit for extracting crude methanol containing 4 wt% water at about 38°C (100°F). The extractor unit and water wash unit are operated at 35-65°C (100-150°F) and 100-2000 kPa. The methanol distillation tower operates at about 100-350 kPa with overhead at about 35-95°C and bottoms at about 120-150°C. The stream composition for each feed, light extract phase and heavy raffinate phase is given in Table I.

## Table 1. Extraction Operation

| Component | FCC Light Naphtha | Crude Methanol | Raffinate Light Liquid phase | Raffinate Heavy liquid Phase |
|---|---|---|---|---|
| Methanol (1bmol/hr) | | 149.87 | 113.96 | 35.91 |
| Water | | 11.11 | 0.40 | 10.71 |
| $C_4$ | 51.13 | | 50.98 | 0.15 |
| $C_5$ | 330.10 | | 329.23 | 0.87 |
| $C_6$ | 163.38 | | 163.02 | 0.36 |
| Total | 544.61 | 160.98 | 657.59 | 48.00 |

Methanol Recovered (wt%)          76.0

Water Entrained in Methanol          0.2*

(*based on dry hydrocarbon feed)

Etherification Operation

The reaction of methanol with isobutylene and isoamylenes at moderate conditions with a resin catalyst is known technology, as provided by R. W. Reynolds, et al., The Oil and Gas Journal, June 16, 1975, and S. Pecci and T. Floris, Hydrocarbon Processing, December 1977. An article entitled "MTBE and TAME - A Good Octane Boosting Combo", by J. D. Chase, et al., The Oil and Gas Journal, April 9, 1979, pages 149-152, discusses the technology. A preferred catalyst is a trifunctional ion exchange resin which etherifies, hydrogenates and isomerizes the reactant streams. A typical acid catalyst is Amberlyst 15 sulfonic acid resin.

MTBE and TAME are known to be high octane ethers. The article by J. D. Chase, et al., Oil and Gas Journal, April 9, 1979, discusses the advantages one can achieve by using these materials to enhance gasoline octane. The octane blending number of MTBE when 10% is added to a base fuel (R + O = 91) is about 120. For a fuel with a low motor rating (M + O = 83) octane, the blending value of MTBE at the 10% level is about 103. On the other hand, for an (R + O) of 95 octane fuel, the blending value of 10% MTBE is about 114.

Processes for producing and recovering MTBE and other methyl tertiary alkyl ethers for $C_4$-$C_7$ isoolefins are known to those skilled in the art, such as disclosed in U.S. Patents 4,544,776 (Osterburg et al) and 4,603,225 (Colaianne et al). Various suitable extraction and distillation techniques are known for recovering ether and hydrocarbon streams from etherication effluent.

The present invention is particularly advantageous in the economic dewatering of crude methanol, thus avoiding expensive and energy-intensive prefractionation by distillation. By extracting methanol from the crude feedstock with hydrocarbon reactant, distilling the aqueous raffinate and methanol recycle streams together in a single distillation tower, substantial utilities and equipment savings are realized.

Various modifications can be made to the system, especially in the choice of equipment and non-critical processing steps. While the invention has been described by specific examples, there is no intent to limit the inventive concept as set forth in the following claims.

## Claims

1. A continuous process for converting crude methanol to methyl tertiary alkyl ethers in a catalytic reaction zone with acid etherification catalyst comprising the steps of:

(a) contacting a primary crude methanol feedstock stream containing a minor amount of water with a liquid hydrocarbon extraction stream rich in $C_4^+$ isoalkene hydrocarbons under extraction conditions favorable to selective extraction of the methanol, thereby providing an extract liquid stream rich in methanol and an aqueous raffinate stream lean in methanol;

(b) charging the extract liquid stream rich in methanol to the catalytic reaction zone under process conditions for converting methanol to predominantly methyl tertiary alkyl ether;

(c) distilling the aqueous raffinate stream to recover a secondary methanol stream and residual hydrocarbon for reaction in step (b);

(d) contacting the reaction product from step (b) with water to wash methanol therefrom and thereby provide a product stream rich in methyl teritrary alkyl ether; and

(e) further fractionating the methanol-rich wash water from step (d) together with the raffinate in step (c).

2. The process of claim 1 wherein the acid catalyst comprises ion exchange resin and the wash water comprises at least a portion of the fractionated raffinate water recovered from distillation step (c).

3. The process of claim 1 or 2 wherein the feedstock consists essentially of methanol and 4 to 20 wt% water, and wherein the extraction liquid comprises a major amount of $C_4$-$C_5$ isoalkenes.

4. The process of claim 1, 2 or 3 wherein $C_4$ + hydrocarbons employed in the extraction step (a) comprise olefinic crackate light naphtha from a fluidized catalytic cracker.

5. A process for converting crude aqueous methanol feedstock to methyl tertiary alkyl ether product in contact with acid catalyst comprising the steps of:
contacting the aqueous methanol feedstock with a liquid hydrocarbon extractant comprising $C_4$ mixed olefinic hydrocarbons comprising isobutylene, n-butenes, and butanes under liquid extraction conditions;

recovering an aqueous raffinate phase containing the major amount of water introduced with the feedstock;

recovering an organic extract phase comprising the hydrocarbon extractant and a major amount of methanol introduced in the feedstock; and

reacting the extracted methanol with the isobutylene under catalytic etherification reaction conditions to produce predominantly methyl t-butyl ether.

6. The process of Claim 5 including the further steps of separating conversion reaction effluent to recover a $C_4^+$ liquid recycle stream comprising isobutane and other $C_4^+$ aliphatic hydrocarbons and an ether product stream; and recycling at least a portion of the $C_4^+$ hydrocarbon stream recovered from the reaction effluent as extractant.

7. The process of Claim 5 or 6 wherein the catalyst consists essentially of sulfonic acid resin and the crude feedstock contains about 2 to 30 wt % water.

8. A continuous feedstock separation and etherification reactor system for converting crude methanol feedstock to methyl t-alkyl ether comprising:

extractor means for contacting crude feedstock liquid containing a minor amount of water with a liquid hydrocarbon extraction steam under extraction conditions favorable to selective extraction of methanol, thereby providing an extract liquid stream rich in methanol and an aqueous raffinate stream lean in methanol;

raffinate separation means for recovering a raffinate methanol stream;

catalytic reactor means operatively connected for contacting the extract stream and raffinate methanol stream in a catalytic reaction zone with acid etherification catalyst in an etherification reaction zone under process conditions to convert a major portion of methanol to ether;

raffinate separator means for distilling the aqueous raffinate stream to recover methanol and residual hydrocarbon for reaction in the catalytic reactor;

effluent washer means for contacting etherification reaction effluent with a portion of fractionated raffinate water recovered from the rafffinate separator to wash methanol from the effluent and thereby provide a product stream rich in methyl isoalkyl ether; and

means for passing methanol-rich wash water from the effluent washer for codistillation with raffinate.

9. The system of claim 8 wherein the extractor means comprises a countercurrent liquid-liquid extraction column.

10. The system of claim 8 or 9 further comprising stabilizer means operatively connected between the reactor means and the effluent washer means for removing light gas from etherification reactor effluent.

FIG. 1

0 289 232

# FIG. 2

### METHANOL RECOVERY VS. CARBON NUMBER OF ALIPHATIC HYDROCARBON: (METHANOL + WATER) RATIO OF 4:1

# FIG. 3

WATER ENTRAINMENT VS. CARBON NUMBER OF ALIPHATIC
HYDROCARBON: (METHANOL + WATER) RATIO OF 4:1